# EUROPEAN PATENT SPECIFICATION

(11) **EP 1 210 990 B1**
(45) Date of publication and mention of the grant of the patent: **22.07.2009**
(21) Application number: 01830743.9
(22) Date of filing: 03.12.2001
(51) Int. Cl.: B09C 1/10, B01F 7/04

(54) **Method for the treatment of soils polluted by heavy metal compounds and related plant of treatment**
Verfahren und Vorrichtung zur Behandlung von durch Schwermetallverbindungen verseuchten Böden
Procédé et installation pour le traitement de sols pollués par des composés de métaux lourds

(30) Priority: 01.12.2000 IT RM000631; 01.12.2000 IT RM000632
(43) Date of publication of application: 05.06.2002
(73) Proprietor: Atzwanger, Michael, 39100 Bolzano (IT); Schinner, Franz, 6080 Innsbruck (AT); Huber, Walter, 39100 Bolzano (IT); Klauser, Thomas, 6020 Innsbruck (AT)
(72) Inventor: Schinner, Franz, 6080 Innsbruck (AT); Huber, Walter, 39100 Bolzano (IT); Klauser, Thomas, 6020 Innsbruck (AT)
(74) Representative: Manfrin, Marta

(56) References cited:
- US-A- 3 845 939
- US-A- 5 458 747
- US-A- 5 824 541
- US-A- 5 849 567
- PATENT ABSTRACTS OF JAPAN vol. 2000, no. 11, 3 January 2001 (2001-01-03) -& JP 2000 210652 A (KOTOO:KK), 2 August 2000 (2000-08-02)

## Description

The present invention concerns a method for the treatment of soils polluted by heavy metal compounds, in particular oxygenated heavy metal compounds, specifically compounds of copper, zinc, tin, mercury, lead, nickel, chromium and the like.

Incidentally, all metals comprised in groups III to XVI, or from group 3A to 6B and in the periods IV (4) and higher of the periodic table of elements are considered to be 'heavy'.

It has long been known that soils may be contaminated both by organic pollutants, like agricultural chemicals, pesticides etc., as well as by inorganic ones, like heavy metal compounds.

Likewise, it is known that pollutants are toxic to soil micro-organisms, inhibiting the specific fertility thereof, and that, via in-plant adsorption, the former enter the human food chain triggering subtle and not always conjecturable damages and illnesses.

Another even more subtle drawback is that both organic and inorganic pollutants, due to soil washout by rain waters which most of the times are acid due to the presence of NOₓs and SOₓs, can reach and poison water beds, with catastrophic ecological effects.

It is known to those skilled in the art that traditional remediation methods mainly comprise:
1. Removing and dumping the soil. This intervention is feasible for small soil areas only (e.g., an 1 ha area for a 50 cm depth equals to about 7.000 ton of soil to be treated) with the likewise need to remediate dumped soil.
2. Converting the soil to other aims (e.g., housing rather than cultivation), yet this intervention is not always viable. Anyhow, over time the pollutants, above all the inorganic ones, may reach the water beds or react, surface-poisoning the environment, above all in the presence of compounds of mercury, lead, chromium, etc.
3.Treating by chemico-physical processes. These procedures can be extremely costly, and anyhow destroy also the beneficial substances present in the soil, in fact rendering the latter unproductive. Anyhow, it is apparent to all that the reactants should be disposed of applying costly procedures. Likewise, apparently post-intervention a soil proves substantially "dead", as no active organic substances and micro-organisms subsist therein.

Among known treatments, there are also those using micro-organisms to get rid in particular of the heavy metal compounds (compounds of copper, tin, mercury, lead, nickel, chromium, etc.). That taught in A. Syed et al., Bioremediation Journal 2(3&4):183-190 (1998) is particularly effective. Therewith, a micro-organism (Aspergillus niger) fed on a completely synthetic substrate containing sucrose as carbon source and ammonium sulfate as nitrogen source, is used. This choice, besides entailing high costs, may cause contamination problems, as this soil is not selective and it can be metabolised by other micro-organisms as well, with a disadvantageous decrease in the fertility of the soil which is rendered unproductive, or, at best, scarcely productive. Moreover, said taught treatment may be carried out merely onto the soil surface, and this, besides facilitating contamination by micro-organisms other than the Aspergillus, entails the drawback of causing the development of preferential channels for the fluid, with the ensuing decrease in the yield of the method. Aspergillus niger is a micro-organism active solely onto the soil surface, therefore in order to thoroughly decontaminate the soil down to a predetermined depth a mechanised ploughing exposing the sublayers is required. Understandably, all this entails remarkable operative disadvantages, as well as relevant costs.

The technical problem underlying the present invention is that of providing a method for the treatment of polluted soils allowing to overcome the drawbacks mentioned with reference to the known art.

This problem is solved by a method as set forth in the independent claim 1.

In fact, it has surprisingly been found by the Inventors that the same heterotroph micro-organisms present in the soil to be remediated, prior to the growth thereof on a suitable and specific medium, may advantageously be used. This treatment transforms the existing and scarcely water-soluble heavy metal compounds into soluble complexes thereof within a wide pH range encompassing typical agricultural soil pHs. The process may take place in tank reactors, or using the same polluted environment whose subsoil have impermeable strata apt to channel the aqueous solutions of the heavy metal complexes thus extracted.

The present invention will hereinafter be disclosed according to a preferred embodiment thereof, along with some preferred embodiments thereof, given by way of example and not for limitative purposes with reference to the following examples and to the attached drawings, wherein:

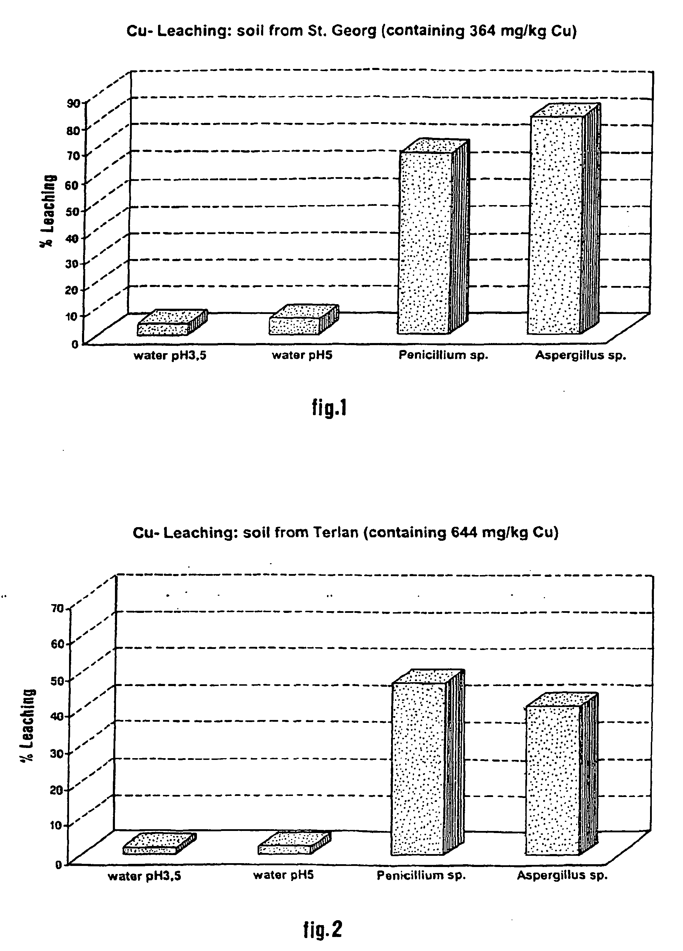

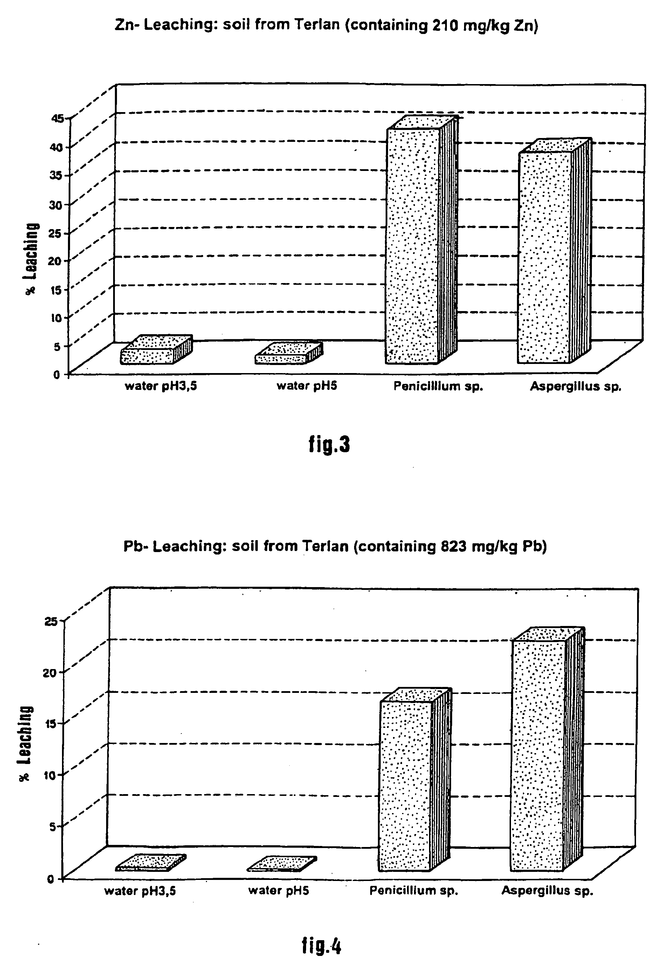

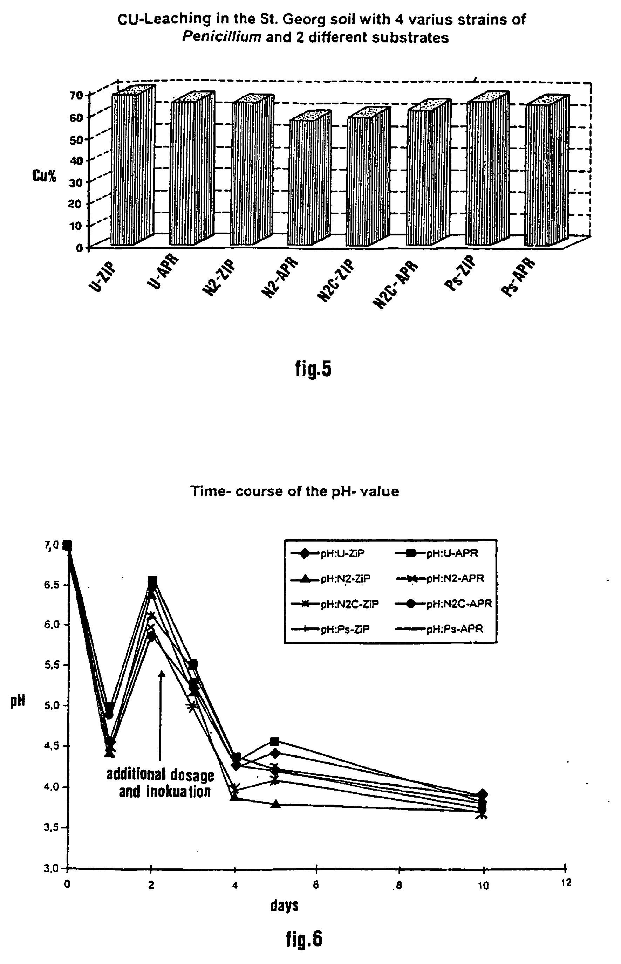

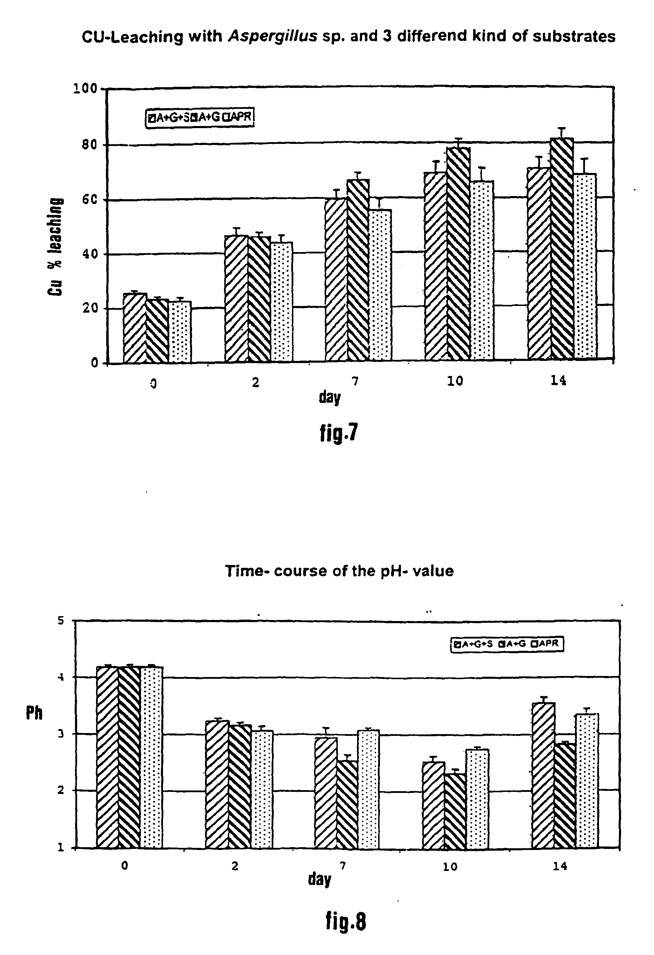

- Figs. 1 to 8 show illustrative diagrams related to the following examples.

The present invention is based on a method capable of extracting heavy metal compounds (like copper, tin, mercury, lead, nickel, chromium etc.) from soils containing them using the same micro-organisms present therein, which are grown using food industry by-products and/or residues and suitable mixtures thereof. In particular, by-products and/or residues of cereals, fruit peels in general and specifically apple and citrus peels are used. These residues may be employed tel quel, minced and dried. Another peculiar feature of the invention lies in that the micro-organisms are fed with sugary substances, in particular deriving from sugar mill waste. More in particular, molasses having a lower sugar content are also employed; the latter are very advantageous, as they are also nitrogen carriers, besides being carbon carriers, with the advantage of concomitantly obtaining a depollution and an enrichment of the treated soil.

Hence, the method according to the present invention is based on the capability of the heterotroph micro-organisms, in particular of the fungi of the Penicillium and Aspergillus genera, in presence of the abovementioned edible materials, of producing substances apt to complex the compounds of the heavy metals present in the soil, and to make them water-soluble. This, according to Werner coordination theory, means that the joint action of the abovementioned micro-organisms and of the edible materials forms binders capable of directly coordinating to the metal, thereby making the latter water-soluble.

The process according to the present invention is based on the capability of the heterotroph micro-organisms, in particular of the fungi of the Penicillium and Aspergillus genera, to hydrosolubilize the heavy metals present in the soil as metal compounds. The process takes place in an aerobic environment, using food industry products, by-products and/or residues, and suitable mixtures thereof, as substrate. In particular, cereals, fruit peels in general, specifically like apple and citrus peels and suitable mixtures thereof, are used. These residues may be employed tel quel, minced, and needwise also dried.

In the method advanced, food residues, in particular fruit residues, are used as a carbon source, whereas as a nitrogen source sugar production wastes, in particular the well-known molasses, are used. These products and/or suitable mixtures thereof, used as culture substrates for growing the micro-organisms, prove to be surprisingly selective therefor, allowing the growth solely of those exhibiting a certain pectinolytic activity. The process may take place on-farmyard in tank reactors under controlled conditions, or it may be applied on site. In the latter case, advantageously the subsoil in the deeper strata (about 1 linear meter) will have impermeable strata apt to channel the aqueous solutions of the polluting heavy metal complexes. Incidentally, the micro-organisms and the employ of the edible materials are suitable to the remediation not merely of soil surfaces, but also of soil bodies. Thus, the percolate mixture substantially regenerates the entire layer below the surface of the polluted soils.

The micro-organisms are selected from the ground, therefore being innocuous thereto, and then cultured onto the abovementioned substrate. These micro-organisms make the heavy metal compounds water-soluble by complexing the latter at a pH similar to the natural pH of the soil. The pH obtained under the micro-organism growth conditions ranges from 2 to 10, more typically from 2.5 to 7. Surprisingly, the heavy metal complexes thus formed are water-soluble within a wide pH range, and may be reclaimed from the washout waters of the process; this requires suitable draining conditions to be contrived process- and soilwise.

The reaction is completed within a time interval ranging from 7 to 14 days, depending on the nature of the soil, and of the type and nature of the heavy metal compound present therein.

With the methodology described in the present invention, decreases of up to 90% or more, with respect to the initial concentration of the heavy metal compound at issue, may be attained. The fluids related to the carrying out of the process, as it will hereinafter be described, are preferably added using a percolation technique, and are continually removed by filtering or draining, or other alike techniques, so as to prevent the heavy metal compounds present therein from reaching levels toxic to the micro-organisms themselves.

The process may advantageously be discontinued for a time period of about one week, and then repeated needwise. The heavy metal complexes dissolved in the filtered or drained liquid may be reclaimed according to techniques known to those skilled in the art (F. Basolo and R. G. Pearson Mechanisms of substitution reactions of metal complexes" in H. J. Emeleus and A. G. Shape ed. and J.C. Bailar, Jr., The Chemistry of Coordination Compounds, Reinhold, New York, 1956 ed.).

The micro-organisms used are heterotroph fungi developing in an aerobic environment. E.g., they are of the genera Penicillium sp. or Aspergillus sp., and originally are in the polluted soil or could be taken from an alike soil having similar features. In said soil, also other fungi already present, of likewise metabolic capabilities, may remain active.

According to the process of the invention, the micro-organisms are isolated from the polluted soil or from an alike soil, then cultured and grown in Laboratory, using the abovementioned edible substrates. These isolating and selecting steps are peculiar and crucial; selection and isolation enable an optimum fungal adaptation to the soil to be treated. These steps are carried out starting from the isolating step:
* Micro-organisms are isolated using standard solutions, like, e.g., CaCl, Ringer solutions, etc., then applied on an agar-selective culture substrate, containing, e.g., CuO or other heavy metal compounds, or, needwise, also antibiotics for selecting the fungi from the bacteria (Raper, K.B. and Fenell, D.J.: the genus Aspergillus (The Williams a. Wilkins Comp., Baltimore, 1965; Warcup, J.H.: The soil-plate method for isolation of fungi from soil - Nature, 1950, 166, pp. 117-118; E. Merck, Mikrobiologisches Handbuch, Darmstadt; Naeveke R., and K.P. Tepper 1979: Einfuehrung in die microbiologischen Arbeitsmethoden, Gustav Fischer Verlag Stuttgart: pp.208 and Srasser, H.: Uebungen zur Geomikrobiologie, Praktikumsanleitung, Institut fuer Mikrobiologie, Universitaet Innsbruck, 1998).
* The selected suitable fungi are first assessed for their capability of growing onto the selective medium and then identified and classified according to the methods indicated hereinafter (J.I. Pitt: The Genus Penicillium and its teleomorphic states Eupenicillium and Talaromyces, Academic Press, Inc., London, 1997; R.A. Samson and J.I. Pitt: Modern Concepts in Penicillium and Aspergillus Classification, NATO ASI Series, series A: Life Sciences vol. 185, Plenum Press New York, 1990; K.H. Dornsch and W. Gams: Compendium of soil fungi; IHW - Verlag Eiching, 1993; K.B. Raper and C. Thom: A Manual of the Penicillia, Williams & Wilkins, Baltimore, 1949; J. Tuite: Aspergillus, Mycotoxins-Mycotoxicoses: An Encyclopedia Handbook, Marcel Dekker, New Dekker, New York, 1977 and M. Rechcigl Jr. CRC Handbook Series in Nutrition and Food, Section G: Diets, Culture Media, Food Supplements; Volume 3, CRC Press; Inc., 1978).
* A suitable amount of medium comprising food residues is sterilized, e.g., with steam under a 1 bar pressure and at 121°C.
* Preferably, the food residues are, e.g., cereals, like wheat or rye, a amount ranging from 5 to 10% by weight of fruit residues tel quel and/or also dried and minced (see references on the preceding page) being added thereto.
* Onto this substrate the inoculation of the spores of the fungi selected according to the methodologies of the preceding page is carried out. After a 6-10 day interval the inoculated substrate is submersed in a KCl solution, preferably at a concentration of about 10g/l, and stirred for about one hour; about 200 ml of the resulting solution are used for a culture of 30g sterilized cereals with 30 ml water.
* Thus, inoculated food residues, as well as a fungine spore solution are obtained; both may be used for in-soil inoculation.

The treatment of soils according to the invention is preferably carried out as follows.

The soil to be treated is mixed to the food residues, inoculated according to the abovementioned procedure, or, alternatively, with the sporiferous suspension. Optionally, to this mixture structural materials, like, e.g., wooden shavings or the like, apt to provide structure and to facilitate aeration, may be added.

Regardless of where the process takes place (reactor or farmyard), the soil is milled and riddled, e.g., until attaining a <2 mm size.

Preferably, if the process is carried out in a reactor or in a farmyard, the soil is riddled, e.g., to a <2mm size.

The process is started adding to the mixture (soil + inoculated substrate + optional structural material) (the need to employ structural materials depends on the soil type, structure and texture) a sugary aqueous solution, e.g., molasses, whose minimum sugar content should be >40% by weight (b/w).

In this step the optimum oxygen contribution is of about 18 Normal 1/min of air per Kg of mixture; this amount may vary depending on the soil type, on the average size and water content thereof. Therefore, the mixture should be kept damp by water and/or sugary solution contribution, and under constant aeration, e.g., using air injected and/or sucked from the bottom (where possible). The suction is advantageous also to the removal of the solution containing the solubilized metals.

In general, the quantities of food residues and sugary solution with respect to the soil depend on the type and on the features of the soil to be treated. Therefore, the quantities provided for the employ may not be pinpointed, yet may adequately be assessed by a person skilled in the art who merges his/her personal knowledge with the indications of the present invention. The amount varies from soil to soil. However, within certain ranges, the related edible materials/sugary solution ratios remain unchanged.

Preferably, for Penicillium the food residues (dry matter)/molasses (about 65% dry matter) weight ratio is of about 1:1 (sum of the entire process). The total amount of residues is equally divided into three fractions, and it is applied thrice during the process. 10% molasses at the start, and 45% molasses in each of the two initial days of treatment are applied. Molasses is purchased in liquid phase with 65% dry matter and it is water-diluted at a concentration of about 150 g/l.

Preferably, for Aspergillus the food residues (dry matter)/ molasses (about 65% d.m.)/glucose syrup dry weight ratio is of about 10:1:5 (sum of the entire process). The total amount of residues is equally divided into three fractions and applied thrice during the process. Molasses is applied single-dose at the start and glucose syrup is subdivided and applied in three doses, like the residues. Molasses is purchased in liquid phase with about 65% dry matter and it is water-diluted at a concentration of about 150 g/l.

E.g., the process may be carried out:
- In a percolation reactor (e.g. of the vertical, horizontal or rotary drum type).
- In farmyard or aerated mound, on a suitable non-permeable foundation, anyhow enabling percolate collection, e.g., by a sloping support and percolate collecting pipes. To aerate, drilled tubes, and needwise drainage tubes, i.e. tubes commonly used in agriculture and commercially available in various shapes and sizes, may be used. Irrigation is carried out by microjet systems commonly used in fruit-farming cultures.
- On site, with no soil removal, and using a percolation system with irrigation and draining for removing the water containing the solubilized metal ions.
- In a reactor, keeping the soil in an aqueous suspension by means of a suitable stirring system.

The solubilized metal complexes may be removed from the reaction area washing them away to deeper strata in case of an in situ reaction, when the conditions of the water beds and the nature of the heavy metals allow that. In absence of these conditions, the solubilized metal complexes should be eliminated from the reaction area, e.g. by a suitable draining system, collected in suitable containers and specifically disposed of.

The process has preferential conditions according to the type of micro-organism used.

E.g., as abovementioned, Penicillium-type fungi require a high content of molasses at the start of the process and in the two subsequent days. The diluted molasses (about 150 g/l) should percolate slowly (about 100 ml/h per kg of soil) through the soil body.

Periodically (about every 90 min.) and intermittently air is injected and/or vacuum is applied to remove the aqueous solution from the soil and to aerate the reaction area. The percolation treatment is limited in time (10 to 16 hours) and then it is discontinued for that day. A stop of about 8 hours is important for an unperturbed fungal growth. After percolation the soil is washed out with water or with a -0.01 M CaCl₂ solution. ≤800 ml/Kg soil concentrations may be used. The chloride may be replaced by other compounds having analogous ion exchange capabilities for the metal ions; all of the alkali metal and non-harmful alkali earth chlorides may be employed.

Needwise, and depending on the biomass development, the treatment is followed by a 24-hour rest, or by at least an 8-hour stop for unperturbed fungal growth. Every 3-4 days, needwise, i.e., depending on the amount of the already solubilized metals and of the residual metals not yet metabolised by the soil, a new dose of food residues, as abovementioned, is admixed to the soil to be treated. Optionally, also a further inoculation with liquid-phase spores (microbial charge comprised in the range 10⁵-10⁸ spores/ml) is carried out.

During this time interval, the pH of the percolate decreases to values 2-5, and more typically 3-5, soilwise, then tends to rise back to the starting pHs of the treated soil. The peculiarity of this treatment is that anyhow the heavy metal complexes formed are soluble in an aqueous environment and are not overly affected by the pH variation.

This treatment is followed by a resting phase of at least 7 days, during which the soil may also be dried. Then the process may be repeated again. As fruit residues for the Penicillium species, apple, as well as lemon and orange residues are particularly suitable.

Overall, the Aspergillus species requires less molasses with respect to the Penicillium. Molasses is employed in a single-dose, as abovementioned, and metabolised only for about 1 hour. Molasses is employed only once. A slow percolation of the molasses solution is not required, however preferably the molasses should be homogeneously spread in the soil, in order to limit the contact time of the fungus therewith. Otherwise, the fungus produces overabundant biomass, hinders aeration and fails to produce the acids suitable for an appropriate treatment. The first dose of molasses is left for about 2 hours in the soil prior to the first washing out. The subsequent doses are left in-soil from 25 to 60 minutes prior to being washed out. Thus, the fungus spreads everywhere it finds the molasses substrate, grows to the consumption of the latter and optimally produces the acid combination in order to yield the maximum extraction as polluting heavy metal complex. Of course, depending on the type of polluting mental compound, a suitable optimisation of the extraction system is required.

The soil is washed away with water or CaCl₂, preferably 0.01 M, or with other alike product, and it may also be dried. After this treatment the soil is rested for 2-3 days under light aeration. Then a new dose of edible residues, as abovementioned, is admixed, and the soil is aerated. Needwise, in order to rectify the pH via glucose breakdown by the fungi, to the edible residues a small amount of glucose may be added. The pH should reach the lowest possible pH, preferably from 2 to 4, so as to have the fungus produce acids suitable for the heavy metal complexing. These acids depend on the soil type, on the pH and on the physiologic state of the fungus. In particular the acids in charge of the so-called 'leaching' are: citric acid (Cu, Zn), (pK1 = 3.13; pK2 = 4.76; pK3 = 6.40) oxalic acid (pK1 = 1.27; pK2 = 4.27) (Pb), (Pb C₂O₄ pKs=11) malic, fumaric, tartaric, gluconic, ketoglutaric acid. The balance thereamong depends on the pH and it may be managed by suitably varying the pH.

It should be taken into account that the solubility of the complexes depends, apart on the pH, on the value of the complexing constant. Since most of the times the complex breakdown products are scarcely soluble, the importance of complex-forming reactions in the subsequent dissolving of the precipitates or in preventing the formation thereof is apparent. This determines the importance of the intrinsic value of the complexing constants, whose value determines the minimum concentration required to have a precipitation in a determined environment (F.G.C. Rossotti and H. Rossotti, The Determination of Stability Constants, Mc Graw-Hill, New York, 1961).

Subsequently the soil is aerated and rested for 2-3 days. The treatment is characterised by 2 to 4 additional dose cycles with edible residues. The entire procedure may be repeated. Apple residues are highly suitable to Aspergillus.

The process of the invention also applies to a soil or soils polluted by one or more heavy metals, even in the presence of other organic pollutants.

The former may also be conducted on thermal treatment residues, ashes and/or purification sludges. Thus, metal concentration is abated to non-toxic levels, thereby enabling a more cost-effective disposal of these materials, and optionally the use thereof.

With the soil remediation process according to the present invention the soil keeps its original features, the treatment is mild and does not inactivate the organic substances of the soil, which may be reused for agricultural production. Moreover, besides being decisive and effective, the treatment is also cost-effective, as it may be carried out so as to act in depth and it enables the reuse of food industry waste.

Lastly, the method is applied in the body of the treated soil, and not merely onto the surface thereof, thereby also overcoming the problem of the development of preferential flow channels which adversely affect the process performance.

The present invention will hereinafter be described in relation to non-limiting illustrative examples of the scope thereof, making reference to the attached figs. 1 to 8.

### EXAMPLES

In the examples, 500 ml vacuum percolators were used. The soil was vineyard soil, containing 287 mg/kg Cu; the soil was riddled into two fractions: a) <2 mm, b) 2-4 mm. The two fractions were admixed again at the following ratios: 80% a)/ 20% b). The amount of soil employed in each percolator was of 150 g. Micro-organisms Penicillium and Aspergillus, respectively, were used.

### Example 1 - Decontamination with Penicillium sp

### Materials used

Fungus: Penicillium sp isolated from lemon peels
inoculum: 10 spores/ml sporiferous suspension
Structure material: 10% b/w wooden shaving, dried with respect to the soil
Complex nutrient source: 10% b/w ZiP (dried and powdered lemon peels dried and powdered lemon peels) dried with respect to the soil
Sugary nutrient source: 150g/l molasses (65% dry matter), (provided by AGRANA, Ltd.).

### Procedure:

150 g of the soil are admixed to 15 g ZiP and 15 g wooden shaving. This mixture is put in the percolator. Then, a mixture of 40 ml sporiferous suspension, 50 ml molasses solution (150 g/l) and 60 ml CaCl₂ (10 g/l) is added to the soil. In order to initiate percolation, the peristaltic pump (flow of about 15 ml/h per percolator) is started. Needwise, percolation is carried out either with the molasses solution or with pure water. Aeration is implemented either applying a slight vacuum from the bottom of the percolator or providing a slight pressure thereto. Therefore, the procedures follows the scheme shown in Table 1 (in the table, for "Pump start" the starting of the peristaltic pump in order to start percolation is meant).

The inoculation is carried out with the sporiferous suspension, the quantities are indicated in Table 1. At day +8, after the sampling and the subsequent analysis of the heavy metal content, a 3-day soil drying phase ensues, then the soil is extracted from the percolator, minced and reintroduced therein.

**Table 1**

| **Day No** | **Hour** | **Step** | **Percolating solution** | **Aeration** | **Irrigation (with CaCl2)** |
|---|---|---|---|---|---|
| 0 | 20.00 | Pump start | molasses | every 90' for 20' | |
| 1 | 10.00 | Percolation stop | | | |
| | 10.10 | Irrigation | | | 100 ml |
| | 10.15 | Vacuum | | for 5' | |
| | | application | | | |
| | | Sampling | | | |
| | 19.00 | Pump start | molasses | every 90' for 20' | |
| 2 | 10.00 | Percolation stop | | | |
| | | | | | |
| | 10.10 | Irrigation | | | 100 ml |
| | 10.15 | Vacuum application | | for 5' | |
| | | Sampling | | | |
| | 16.00 | Inoculation (10 ml /percolator) | | | |
| 3 | 19.00 | Pump start | water | every 90' for 20' | |
| 4 | 10.00 | Percolation stop | | | |
| | 10.10 | Irrigation | | | 100 ml |
| | 10.15 | Vacuum application | | for 5' | |
| | | Sampling | | | |
| | 13.00 | Additional dose of 10% ZiP (15 g/percolator) | | | |
| | 17.00 | Inoculation (10 ml/percolator) | | | |
| | 18.00 | Addition of 30 ml water / percolator | | | |
| | 20.00 | Compressed air | | every 90' for 4' | |
| 5 | 17.00 | Irrigation | | | 100 ml |
| | 19.00 | Pump start | water | every 90' for 20' | |
| 6 | 10.00 | Percolation stop | | | |
| | 10.10 | Irrigation | | | 100 ml |
| | 10.15 | Vacuum application | | for 5' | |
| | | Sampling | | | |
| | 19.00 | Pump start | water | every 90' for 20' | |
| 7 | 10.00 | Percolation stop | | | |
| | 10.10 | Irrigation | | | 100 ml |
| | 10.15 | Vacuum application | | for 5' | |
| | | Sampling | | | |
| | 19.00 | Pump start | 75 g/l molasses | every 90' for 20' | |
| 8 | 10.00 | Percolation stop | | | |
| | 10.10 | Irrigation | | | |
| | 10.15 | Vacuum application | | for 5' | |
| | | Sampling | | | |
| | 15.00 | Drying | | | |
| 11 | 10.00 | Mincing | | | |
| | 17.00 | Irrigation | | | 250 ml |
| | 18.00 | Vacuum application | | for 5' | |
| | | Sampling | | | |
| 12 | 10.00 | Additional dose of 10% ZiP (15 g/percolator) | | | |
| | 17.00 | Inoculation (10 ml/percolator) | | | |
| | 17.30 | Dose of 70 ml molasses solution (300 g/l) | | | |
| | 19.00 | Pump start | water | every 90' for 20' | |
| 13 | 10.00 | Percolation stop | | | |
| | 10.10 | Irrigation | | | 100 ml |
| | 10.15 | Vacuum application | | for 5' | |
| | | Sampling | | | |
| | 19.00 | Pump start | water | every 90' for 20' | |
| 14 | 10.00 | Percolation stop | | | |
| | 10.10 | Irrigation | | | 100 ml |
| | 10.15 | Vacuum application | | for 5' | |
| | | Sampling | | | |
| | 19.00 | Pump start | water | every 90' for 20' | |
| 15 | 10.00 | Percolation stop | | | |
| | 10.10 | Irrigation | | | 100 ml |
| | 10.15 | Vacuum application | | for 5' | |
| | | Sampling and end of treatment cycle | | | |

| | | | | | |
|---|---|---|---|---|---|
| A >60% elimination is attained with mere two doses of ZiP. | | | | | |

### Example 2 - Decontamination with Aspergillus sp

### Materials used

Fungus: Aspergillus sp isolated from lemon peels
inoculum: 7% inoculated rye
Structure material: wooden shaving, 7% dried with respect to the soil
Complex nutrient source: 10% APR (dried and powdered apple residues) and 5% WT (dried marc) (% dried with respect to the soil)
Sugary nutrient source: molasses (65% dry matter) at the concentration of 150g/l (AGRANA Ltd.) and glucose syrup (about 150g/l).

### Procedure:

150 g of the soil are admixed to 15 g APR, 10 g wooden shaving, 10 g inoculated rye and 10 g WT. This mixture is introduced in the percolator and 100 ml pure water are additioned thereto. The aeration is carried out as in the preceding example.

Hence, the procedure follows the scheme of the following Table 2.

**Table 2**

| **Day No.** | **Hour** | **Step** | **Solution** | **Aeration** | **Irrigation (with CaCl2)** |
|---|---|---|---|---|---|
| 0 | 9.00 | molasses dose | 50 ml | | |
| | 9.30 | Irrigation | | | 200 ml |
| | 10.00 | Vacuum application | | for 15' | |
| | 10.15 | Aeration with pressurized air | | for 60' | |
| | | Sampling | molasses | every 90' for 20' | |
| 2 | 9.00 | Irrigation | | | 200 ml |
| | 9.30 | Vacuum application | | for 15' | |
| | 9.45 | glucose syrup addition | 50 ml | | |
| | 10.00 | Irrigation | | | 100 ml |
| | | Sampling | | | |
| | 10.15 | Vacuum application | | for 15' | |
| | 10.30 | Additional dose of 7.5 g apr (5%) | | | |
| | 10.45 | Addition of 50 ml water | | | |
| | 11.45 | Aeration with compressed air | | for 60' | |
| 7 | 9.00 | Irrigation | | | 200 ml |
| | 9.30 | Vacuum application | | for 15' | |
| | 9.45 | glucose syrup addition | 50 ml | | |
| | 10.00 | Irrigation | | | 100 ml |
| | | Sampling | | | |
| | 10.30 | Additional dose of 7.5 g apr (5%) | | | |
| | 10.45 | Addition of 50 ml water | | | |
| | 11.45 | Aeration with pressurized air | | for 60' | |
| 10 | 9.00 | Irrigation | | | 200 ml |
| | 9.30 | glucose syrup addition | 50 ml | | |
| | 10.00 | Irrigation | | | 100 ml |
| | | Sampling | | | |
| | 10.15 | Vacuum application | | for 15' | |
| | 10.30 | Additional dose of 7.5 g apr (5%) | | | |
| | 10.45 | addition of 50 ml water | | | |
| | 11.45 | Aeration with pressurized air | | for 60' | |
| 14 | 9.00 | Irrigation | | | 200 ml |
| | 9.30 | glucose syrup addition | 50 ml | | |
| | 10.00 | Irrigation | | | 100 ml |
| | 10.15 | Vacuum application | | for 15' | |
| | | Sampling | | | |

| | | | | | |
|---|---|---|---|---|---|
| an 82% elimination is attained in a 14-day time period. | | | | | |

### COMPARISON EXAMPLES

Comparison of leaching between aqueous solution at pH 3,5 and pH 5 (HCl-adjusted pH), and leaching with Penicillium and Aspergillus with the hereto disclosed method of the present invention. All this for Cu, Zn, Pb in two soils.

Method: the pH of the aqueous solutions is adjusted with HCl. For each extraction, 5g soil with 100 ml acid solution in a flask is used. Upon stirring, the flask is centrifuged for 1 hour, and the concentration of the metal solubilized in the liquid phase is determined..

Figure 1: Cu solubilization with aqueous solution (pH 3,5 and pH 5) and with "bioleaching" by Penicillium and Aspergillus disclosed in the patent. Soil containing 364 mg/Kg Cu.

Figure 2: Cu solubilization with aqueous solution (pH 3,5 and pH 5), and with "bioleaching" by Penicillium and Aspergillus disclosed in the patent. Soil containing 644 mg/Kg Cu.

Figure 3: Cu solubilization with aqueous solution (pH 3,5 and pH 5), and with "bioleaching" by Penicillium and Aspergillus disclosed in the patent. Soil containing 210 mg/Kg Zn.

Figure 4: Cu solubilization with aqueous solution (pH 3,5 and pH 5), and with "bioleaching" by Penicillium and Aspergillus disclosed in the patent. Soil containing 823 mg/Kg Pb.

### OTHER EXAMPLES

Hereinafter, examples confirming the specific "bioleaching" properties of the micro-organisms Penicillium and Aspergillus are reported.

Figure 5: Cu solubilization in a soil containing 364 mg/Kg Cu. Test with 4 strains of Penicillium: U, N2, N2C, Ps; Employ of 2 substrates deriving from fruit residues: ZiP (dried and minced lemon residues) and APR (dried and minced apple residues). Test with 150g soil in 500 ml percolators. Employ of 10% structural material (wooden shavings), 10% (+ additional 10%) fruit residues.

Figure 6: pH trend. Test with 4 strains of Penicillium: U, N2, N2C, Ps; Employ of 2 substrates deriving from fruit residues; ZiP (dried and minced lemon residues) and APR (dried and minced apple residues). Test with 150g soil in 500 ml percolators. Employ of 10% structure material (wooden shavings), 10% fruit residues plus 10% additional dose thereof.

Figure 7: Cu solubilization from a soil containing 287 g/Kg Cu. Test with 150 g soil in 500 ml percolators. Employ of 15% (by weight of the mixture) fruit residues (dried and minced apple and marc residues) plus molasses (5%) at the start for all variations: Variation of additional dose of 3 different substrates, between days +2 and +7:
A+G+S variation: 5% apple + 5% glucose + 1% soy (always by weight of the mixture)
A+G variation: 5% apple + 5% glucose
APR variation: 5% apple

Figure 8: pH trend. Test with 150g soil in 500 ml percolators. Employ of 15% (by weight of the mixture) fruit residues (dried and minced apple and marc residues) plus molasses (5%) at the start for all variations: Variation of additional dose of 3 different substrates, between days +2 and +7:
A+G+S variation: 5% apple + 5% glucose + 1% soy (always by weight of the mixture)
A+G variation: 5% apple + glucose 5%
APR variation: 5% apple

To the abovedescribed method for the treatment a person skilled in the art, in order to satisfy further and contingent needs, may effect several further modifications and variants, all however falling within the protective scope of the present invention, as set forth in the appended claims.

## Claims

1. A method for the treatment of soils polluted by heavy metal compounds based on the use of micro-organisms, **characterised in that** the micro-organisms are those present in soils and are grown in an aerobic environment on edible materials comprising food industry products, by-products and/or residues, and mixtures thereof, said method comprising the following stops:
* isolating and selecting the soil;
* mixing the soil to be treated to the resulting mixture of micro-organisms and sterilized edible materials;
* keeping the mixture of soil, edible materials and micro-organisms damp, by contribution of water and/or of a sugary solution, and aerated by injecting and/or sucking air, the pH ranging from 2 to 10 being provided and
* draining solutions of heavy metal complexes polluting the treated soil under conditions adequate and suitable to the draining of the solutions of the heavy metal complexes.

2. The method according to claim 1, wherein said pH range is 2, 5 to 7.

3. The method according to claim 1, wherein the edible materials are selected from cereals, fruit peels in general, apple and citrus peels in particular, and mixtures thereof.

4. The method according to claim 3, wherein the edible materials are minced.

5. The method according to claim 3, wherein the edible materials are dried.

6. The method according to claim 1, wherein the edible materials are steam sterilized at temperatures ranging from 100 to 180°C.

7. The method according to claim 6, wherein steam sterilization is carried out at temperatures ranging from 100 to 130°C.

8. The method according to claim 1, wherein the substances contained in the sugary solution are sugar mill wastes.

9. The method according to claim 8, wherein the sugary substances are selected from glucose syrup, molasses and mixtures thereof having glucose contents >20% by weight.

10. The method according to claim 9, wherein the sugary substances are selected from glucose syrup, molasses and mixtures thereof having glucose contents >40% by weight.

11. The method according to claim 1, wherein the micro-organisms are fungi of the genera Penicillium and Aspergillus.

12. The method according to claim 1, wherein the growth is carried out inoculating a amount of edible materials with spores deriving from the selected micro-organisms to which, after a time period ranging from 6 to 10 days, a KCl solution is added and the resulting mixture is placed under stirring so as to obtain inoculated edible materials as well as a fungine sporiferous solution, both useful for inoculation in the soil to be treated.

13. The method according to claim 12, wherein the inoculus is carried out on a amount of material consisting of cereals, the same amount by weight of water and a amount ranging from 5 to 10% by weight of fruit residues.

14. The method according to claim 1, wherein to the mixture of soil, micro-organisms and edible materials structural materials are added, apt to provide structure and to facilitate the aeration thereof.

15. The method according to any one of the preceding claims, wherein the treatment is carried out for a time period ranting from 7 to 14 days.

16. The method according to any one of the preceding claims, wherein liquids, substantially consisting of water and/or of sugary solution, are added by percolation techniques and are continually removed by filtering, draining or other alike techniques.

17. The method according to any one of the preceding claims, wherein the treatment is discontinued for the time period of a week and then it is repeated.

18. The method according to any one of the preceding claims, wherein the micro-organism used is Penicillium, the dry weight ratio between the edible materials and the molasses ranges from 1:0,5 to 1:1,5, the total amount of edible materials being equally subdivided in three factions and applied in three quantities during the treatment, the molasses being used as a water-diluted solution at a 150 g/l concentration.

19. The method according to claim 18, wherein the molasses solution is percolated through the soil at about 100 ml/h per kg of soil; periodically, about every 90 min., and intermittently, air is injected and/or vacuum is applied in order to remove the liquids from the soil and to aerate the latter; the percolation being limited in time from 10 to 16 hours during a day of treatment and then being discontinued for that day; at the end of percolation the soil being washed out with water or with a CaCl₂ solution of about 0,01 M, or alike substance at a concentration of up to 800 ml per kg of soil.

20. The method according to claim 19, wherein the washout is followed by a 24-h rest and then, every 3-4 days, to the soil to be treated another amount of edible material is admixed, and optionally a further inoculation of spores in a liquid phase with a microbial charge ranging from 10³-10¹⁰ spores/ml is carried out, said treatment being followed at least by a 7-day rest, the procedure being repeatable thereafter.

21. The method according to any one of the claim 18 to 20, wherein the edible materials for Penicillium are apple, lemon and orange residues.

22. The method according to any one of the preceding claims, wherein the micro-organism used is Aspergillus, the dry weight ratio among the edible materials, the molasses, and the syrup ranges from 20:1:1 to 1:1:10, the total amount of edible materials being equally subdivided in three fractions and applied in three quantities during the treatment, the molasses being applied in single-dose at the start and the glucose syrup being subdivided and applied in three doses like the edible materials, the molasses being used water-diluted at a 150 g/l concentration.

23. The method according to claim 22, wherein the molasses is homogeneously added to the soil and left to be metabolised by the micro-organisms for a time period of 1 hour, the soil being then washed out with water or about 0,01 M CaCl₂ or alike substance, rested for 2-3 days under light aeration by sucking and/or injecting air, then at least another amount of edible residues is admixed, and the soil is aerated by sucking and/or injecting air.

24. The method according to claim 23, wherein to the edible residues an amount of glucose such that the pH reach the range of 2 to 5 is added.

## Patentansprüche

1. Verfahren zum Behandeln von mit Schwermetallbestandteilen kontaminiertem Boden, basierend auf dem Einsatz von Mikroorganismen, **dadurch gekennzeichnet, dass** die Mikroorganismen solche sind, die in Böden vorliegen und die in einer aeroben Umgebung auf essbaren Materialien gezogen werden, umfassend Nahrungsmittelindustrie-Produkte, Nebenprodukte und/oder Reste und Mischungen derselben, wobei das Verfahren die folgenden Schritte umfasst:
• Isolieren und Auswählen des Bodens;
• Mischen des zu behandelnden Bodens mit der resultierenden Mischung aus Mikroorganismen und sterilisierten essbaren Materialien;
• Feuchthalten der Mischung aus Boden, essbaren Materialien und Mikroorganismen durch Zugabe von Wasser und/oder einer Zuckerlösung und Belüftet halten durch Einleiten oder Saugen von Luft, wobei ein pH in einem Bereich von 2 bis 10 bereitgestellt wird;
• Ablassen der Lösungen der den behandelten Boden kontaminierenden Schwermetallkomplexe unter Bedingungen, die dem Ablassen der Lösungen der Schwermetallkomplexe angemessen und hierfür geeignet sind.

2. Verfahren nach Anspruch 1, wobei der pH-Bereich zwischen 2,5 und 7 liegt.

3. Verfahren nach Anspruch 1, wobei die essbaren Materialien ausgewählt sind aus Getreide, Fruchtschalen im Allgemeinen, insbesondere Apfel- und Citrusfruchtschalen und Mischungen derselben.

4. Verfahren nach Anspruch 3, wobei die essbaren Materialien zerkleinert werden.

5. Verfahren nach Anspruch 3, wobei die essbaren Materialien getrocknet werden.

6. Verfahren nach Anspruch 1, wobei die essbaren Materialien bei Temperaturen in einem Bereich von 100 bis 180°C dampfsterilisiert werden.

7. Verfahren nach Anspruch 6, wobei die Dampfsterilisation bei Temperaturen in einem Bereich von 100 bis 130°C durchgeführt wird.

8. Verfahren nach Anspruch 1, wobei die in der Zuckerlösung enthaltenden Substanzen Zuckerfabrik-Abfälle sind.

9. Verfahren nach Anspruch 8, wobei die Zuckersubstanzen ausgewählt sind aus Glucosesirup, Molassen und Mischungen derselben mit Glucosegehalten von >20 Gewichtsprozent.

10. Verfahren nach Anspruch 9, wobei die Zuckersubstanzen ausgewählt sind aus Glucosesirup, Molassen und Mischungen derselben mit Glucosegehalten von >40 Gewichtsprozent.

11. Verfahren nach Anspruch 1, wobei die Mikroorganismen Pilze der Gattungen *Penicillium* und *Aspergillus* sind.

12. Verfahren nach Anspruch 1, wobei das Wachstum durch Impfen einer Menge an essbaren Materialien mit Sporen durchgeführt wird, die von den ausgewählten Mikroorganismen abgeleitet sind, zu denen nach einer Zeitdauer in einem Bereich von 6 bis 10 Tagen eine KCl-Lösung zugegeben wird und die resultierende Mischung gerührt wird, so dass geimpfte essbare Materialen ebenso wie eine Pilzsporenlösung, die beide zum Impfen von zu behandelndem Boden geeignet sind, erhalten werden.

13. Verfahren nach Anspruch 12, wobei das Impfen mit einer Menge an Material durchgeführt wird, bestehend aus Getreide, derselben Menge an Gewicht an Wasser und einer Menge in einem Bereich von 5 bis 10 Gewichtsprozent and Fruchtresten.

14. Verfahren nach Anspruch 1, wobei zu der Mischung aus Boden, Mikroorganismen und essbaren Materialien Strukturmaterialien zugegeben werden, die geeignet sind Struktur bereitzustellen und die Belüftung derselben zu erleichtern.

15. Verfahren nach einem der vorhergehenden Ansprüche, wobei die Behandlung für eine Zeitdauer in einem Bereich von 7 bis 14 Tagen durchgeführt wird.

16. Verfahren nach einem der vorhergehenden Ansprüche, wobei Flüssigkeiten, die im Wesentlichen aus Wasser und/oder Zuckerlösung bestehen durch Durchsickerungs-Verfahren zugegeben werden und kontinuierlich durch Filtrieren, Ablassen oder ähnliche Techniken entfernt werden.

17. Verfahren nach einem der vorhergehenden Ansprüche, wobei die Behandlung für eine Zeitdauer von einer Woche unterbrochen und dann wiederholt wird.

18. Verfahren nach einem der vorhergehenden Ansprüche, wobei der verwendete Mikroorganismus *Penicillium* ist, das Trockengewichts-Verhältnis zwischen den essbaren Materialien und den Molassen in einem Bereich von 1:0,5 bis 1:1,5 liegt, die gesamte Menge der essbaren Materialien zu gleichen Teilen in drei Fraktionen unterteilt wird und in drei Mengen während der Behandlung angewendet wird, wobei die Molassen als mit Wasser verdünnte Lösung mit einer Konzentration von 150 g/l verwendet werden.

19. Verfahren nach Anspruch 18, wobei die Lösung der Molassen durch den Boden mit ungefähr 100 ml/Std pro kg Boden durchsickert; periodisch, ungefähr alle 90 Minuten, und intermittierend Luft eingeleitet und/oder Vakuum angelegt wird, um die Flüssigkeiten von dem Boden zu entfernen und letzteren zu belüften; wobei die Durchsickerung zeitlich für 10 bis 16 Stunden während eines Behandlungstages beschränkt ist und dann für den Tag unterbrochen wird; wobei am Ende der Durchsickerung der Boden mit Wasser oder mit einer ungefähr 0,01 M CaCl₂-Lösung oder einer ähnlichen Substanz, mit einer Konzentration von bis zu 800 ml pro kg Boden, ausgewaschen wird.

20. Verfahren nach Anspruch 19, wobei die Auswaschung von einer 24 stündigen Pause gefolgt wird und dann alle 3 bis 4 Tage eine weitere Menge essbarer Materialien zu dem zu behandelnden Boden zugemischt wird, und optional eine weitere Impfung mit Sporen in einer flüssigen Phase mit einer mikrobiellen Charge in einem Bereich von 10³ bis 10¹⁰ Sporen/ml durchgeführt wird, wobei die Behandlung von einer wenigstens 7 tägigen Pause gefolgt wird und wobei das Verfahren nachfolgend wiederholt wird.

21. Verfahren nach einem der Ansprüche 18 bis 20, wobei die essbaren Materialien für *Penicillium* Apfel-, Zitronen- und Orangenreste sind.

22. Verfahren nach einem der vorhergehenden Ansprüche, wobei der verwendete Mikroorganismus *Aspergillus* ist, das Trockengewichts-Verhältnis zwischen den essbaren Materialien, den Molassen und dem Sirup in einem Bereich von 20:1:1 bis 1:1:10 liegt, die gesamte Menge der essbaren Materialien zu gleichen Teilen in drei Fraktionen unterteilt wird und in drei Mengen während der Behandlung angewendet wird, wobei die Molassen als Einzeldosis zu Beginn verwendet werden und der Glucosesirup unterteilt wird und ein drei Dosen wie die essbaren Materialien verwendet wird, wobei die Molassen mit Wasser verdünnt mit einer Konzentration von 150 g/l verwendet werden.

23. Verfahren nach Anspruch 22, wobei die Molassen dem Boden homogen zugegeben werden und zum Metabolisieren durch die Mikroorganismen für eine Zeitdauer von 1 Stunde belassen werden, anschließend wird der Boden mit Wasser oder ungefähr 0,01 M CaCl₂ oder einer ähnlichen Substanz ausgewaschen, für 2 bis 3 Tage unter leichter Belüftung durch Saugen und/oder Einleiten von Luft belassen, dann wird wenigstens eine weitere Menge essbarer Reste zugemischt und der Boden wird durch Saugen und/oder Einleiten von Luft belüftet.

24. Verfahren nach Anspruch 23, wobei zu den essbaren Resten eine Glucosemenge derart zugegeben wird, dass der pH einen Bereich von 2 bis 5 erreicht.

## Revendications

1. Procédé pour le traitement de sols pollués par des composés de métaux lourds basé sur l'utilisation de micro-organismes, **caractérisé en ce que** les micro-organismes sont ceux présents dans les sols et qui croissent dans un environnement aérobie sur des matières comestibles comprenant des produits, des sous-produits et/ou des résidus de l'industrie alimentaire, et leurs mélanges, ledit procédé comprenant les étapes suivantes :
- l'isolement et la sélection du sol ;
- le mélange du sol à traiter avec le mélange résultant de micro-organismes et de matières comestibles stérilisées ;
- le maintien à l'état humide du mélange composé du sol, des matières comestibles et des micro-organismes, en utilisant de l'eau et/ou une solution sucrée, et en aérant par injection et/ou par aspiration de l'air, la plage de pH fournie étant de 2 à 10, et
- le drainage de solutions de complexes de métaux lourds polluant le sol traité dans des conditions adéquates et appropriées pour le drainage des solutions des complexes de métaux lourds.

2. Procédé selon la revendication 1, dans lequel ladite plage de pH s'étend de 2,5 à 7.

3. Procédé selon la revendication 1, dans lequel les matières comestibles sont choisies parmi les céréales, les pelures de fruits en général, les pelures de pommes et d'agrumes en particulier, et leurs mélanges.

4. Procédé selon la revendication 3, dans lequel les matières comestibles sont finement hachées.

5. Procédé selon la revendication 3, dans lequel les matières comestibles sont séchées.

6. Procédé selon la revendication 1, dans lequel les matières comestibles sont stérilisées à la vapeur d'eau à des températures de 100 °C à 180 °C.

7. Procédé selon la revendication 6, dans lequel la stérilisation à la vapeur d'eau est réalisée à des températures de 100 °C à 130 °C.

8. Procédé selon la revendication 1, dans lequel les substances contenues dans la solution sucrée sont des déchets de moulin à sucre.

9. Procédé selon la revendication 8, dans lequel les substances sucrées sont choisies parmi le sirop de glucose, les mélasses et leurs mélanges ayant des teneurs en glucose supérieures à 20 % en poids.

10. Procédé selon la revendication 9, dans lequel les substances sucrées sont choisies parmi le sirop de glucose, les mélasses et leurs mélanges ayant des teneurs en glucose supérieures à 40 % en poids.

11. Procédé selon la revendication 1, dans lequel les micro-organismes sont des champignons du genre *Penicillium* et *Aspergillus.*

12. Procédé selon la revendication 1, dans lequel la croissance est réalisée en inoculant une quantité de matières comestibles avec des spores dérivant des micro-organismes sélectionnés auxquels, après une durée de 6 à 10 jours, une solution de KCl est ajoutée et le mélange résultant est placé sous agitation afin d'obtenir des matières comestibles inoculées ainsi qu'une solution sporifère de champignons, toutes deux étant utiles pour l'inoculation du sol à traiter.

13. Procédé selon la revendication 12, dans lequel l'inoculus est réalisé sur une quantité de matière constituée de céréales, la même quantité en poids d'eau et une quantité de 5 à 10 % en poids de résidus de fruits.

14. Procédé selon la revendication 1, dans lequel au mélange du sol, des micro-organismes et des matières comestibles matières de structure sont ajoutées pour fournir une structure et pour faciliter son aération.

15. Procédé selon l'une quelconque des revendications précédentes, dans lequel le traitement est réalisé pendant une durée allant de 7 à 14 jours.

16. Procédé selon l'une quelconque des revendications précédentes, dans lequel des liquides, consistant essentiellement en de l'eau et/ou une solution sucrée, sont ajoutés par des techniques de percolation et sont éliminés en continu par filtration, drainage ou d'autres techniques équivalentes.

17. Procédé selon l'une quelconque des revendications précédentes, dans lequel le traitement est interrompu pendant une durée d'une semaine puis est répété.

18. Procédé selon l'une quelconque des revendications précédentes, dans lequel le micro-organisme utilisé est *Penicillium,* le rapport en poids sec entre les matières comestibles et la mélasse est situé dans la plage allant de 1 : 0,5 à 1 : 1,5, la quantité totale de matières comestibles étant subdivisée en parts égales dans trois fractions et appliquée en trois quantités pendant le traitement, la mélasse étant utilisée à titre de solution diluée dans l'eau à une concentration de 150 g/l.

19. Procédé selon la revendication 18, dans lequel la solution de mélasses est percolée à travers le sol à environ 100 ml/h par kg de sol ; périodiquement, environ toutes les 90 minutes, et de manière intermittente, de l'air est injecté et/ou un vide est appliqué afin d'éliminer les liquides du sol et d'aérer ce dernier ; la percolation étant limitée à une durée de 10 à 16 heures pendant une journée de traitement puis arrêtée pour cette journée ; à la fin de la percolation, le sol est lavé avec de l'eau ou une solution de CaCl₂ à environ 0,01 M, ou avec une substance semblable à une concentration allant jusqu'à 800 ml par kg de sol.

20. Procédé selon la revendication 19, dans lequel le lavage est suivi d'un repos de 24 heures puis, tous les 3 à 4 jours, une autre quantité de matières comestibles est mélangée avec le sol à traiter et, facultativement, une inoculation supplémentaire de spores dans une phase liquide avec une charge microbienne de 10³ à 10¹⁰ spores/ml est réalisée, ledit traitement étant suivi d'un repos d'au moins 7 jours, la procédure pouvant être répétée par la suite.

21. Procédé selon l'une quelconque des revendications 18 à 20, dans lequel les matières comestibles pour *Penicillium* sont des résidus de pomme, de citron et d'orange.

22. Procédé selon l'une quelconque des revendications précédentes, dans lequel le micro-organisme utilisé est *Aspergillus,* le rapport en poids sec entre les matières comestibles, les mélasses et le sirop est situé dans la plage allant de 20 : 1 : 1 à 1 : 1 : 10, la quantité totale de matières comestibles étant subdivisée en parts égales dans trois fractions et appliquée en trois quantités pendant le traitement, les mélasses étant appliquées en une seule dose au début et le sirop de glucose étant subdivisé et appliqué en trois doses comme les matières comestibles, les mélasses étant utilisées diluées dans l'eau à une concentration de 150 g/l.

23. Procédé selon la revendication 22, dans lequel les mélasses sont ajoutées de manière homogène au sol et laissées pendant une durée de 1 heure pour être métabolisées par les micro-organismes, le sol étant ensuite lavé avec de l'eau ou du CaCl₂ à 0,01 M ou avec une substance semblable, laissé au repos pendant 2 à 3 jours sous aération légère par aspiration et/ou injection d'air, puis au moins une autre quantité de résidus comestibles est ajoutée, et le sol est aéré par aspiration et/ou injection d'air.

24. Procédé selon la revendication 23, dans lequel une quantité de glucose est ajoutée aux résidus comestibles de manière à ce que le pH atteigne la plage allant de 2 à 5.
